# EUROPÄISCHE PATENTSCHRIFT

(11) **EP 1 509 611 B1**
(45) Veröffentlichungstag und Bekanntmachung des Hinweises auf die Patenterteilung: **15.08.2007**
(21) Anmeldenummer: 03755083.7
(22) Anmeldetag: 22.05.2003
(51) Int. Cl.: C12N 15/87

(54) **VERFAHREN ZUM TRANSFER VON MOLEKÜLEN IN VITALE ZELLEN MITTELS LASERSTRAHLUNG SOWIE ANORDNUNG ZUR DURCHFÜHRUNG DES VERFAHRENS**
METHOD FOR TRANSFERRING MOLECULES IN VITAL CELLS BY MEANS OF LASER BEAMS AND ARRANGEMENT FOR CARRYING OUT SAID METHOD
PROCEDE DE TRANSFERT DE MOLECULES DANS DES CELLULES VITALES A L'AIDE DU RAYONNEMENT LASER ET SYSTEME PERMETTANT DE METTRE EN OEUVRE LEDIT PROCEDE

(30) Priorität: 23.05.2002 DE 10223922; 23.05.2002 DE 10223921
(43) Veröffentlichungstag der Anmeldung: 02.03.2005
(73) Patentinhaber: König, Karsten, 66119 Saarbrücken (DE)
(72) Erfinder: KÖNIG, Karsten, 66119 Saarbrücken (DE); TIRLAPUR, Uday K. c/o Research Institute, Headington Oxford OX3 7LJ (GB)
(74) Vertreter: Freitag, Joachim
(86) Internationale Anmeldenummer: PCT/DE2003/001708
(87) Internationale Veröffentlichungsnummer: WO 2003/100069

(56) Entgegenhaltungen:
- TIRLAPUR UDAY K ET AL: "Near-infrared femtosecond laser pulses as a novel non-invasive means for dye-permeation and 3D imaging of localised dye-coupling in the Arabidopsis root meristem." PLANT JOURNAL, Bd. 20, Nr. 3, November 1999 (1999-11), Seiten 363-370, XP002257731 ISSN: 0960-7412
- KÖNIG K: "Robert Feulgen Prize Lecture. Laser tweezers and multiphoton microscopes in life sciences." HISTOCHEMISTRY AND CELL BIOLOGY. GERMANY AUG 2000, Bd. 114, Nr. 2, August 2000 (2000-08), Seiten 79-92, XP002257732 ISSN: 0948-6143
- KÖNIG K: "Multiphoton microscopy in life sciences." JOURNAL OF MICROSCOPY. ENGLAND NOV 2000, Bd. 200 ( Pt 2), November 2000 (2000-11), Seiten 83-104, XP002257733 ISSN: 0022-2720
- TIRLAPUR UDAY K ET AL: "Femtosecond near-infrared laser pulses elicit generation of reactive oxygen species in mammalian cells leading to apoptosis-like death" EXPERIMENTAL CELL RESEARCH, Bd. 263, Nr. 1, 1. Februar 2001 (2001-02-01), Seiten 88-97, XP002257734 ISSN: 0014-4827
- TIRLAPUR UDAY K ET AL: "Targeted transfection by femtosecond laser." NATURE. ENGLAND 18 JUL 2002, Bd. 418, Nr. 6895, 18. Juli 2002 (2002-07-18), Seiten 290-291, XP002257735 ISSN: 0028-0836

## Beschreibung

Die Erfindung beinhaltet ein optisches Verfahren zum gezielten Transfer von Molekülen, bevorzugt von DNA, RNA, Peptiden, Aminosäuren und Proteinen, in vitale Zellen mittels Laserstrahlung sowie eine Anordnung zur Durchführung des Verfahrens.
Das Verfahren eignet sich vorzugsweise zur Transfektion von pflanzlichen, tierischen und humanen Zellen, beispielsweise zur Herstellung von Pharmaka, wie synthetische Vakzine.
Mittels der erfindungsgemäßen Anordnung, lässt sich das Verfahren auf effiziente Weise für die Transfektion von Genen in Einzelzellen einsetzen und erschließt Anwendungen auf den Gebieten der Herstellung von Pflanzen- und Tiermaterial, der Gentherapie und der Herstellung und Applikation von speziellen Pharmaka, insbesondere synthetischen Vakzinen.

Der gezielte Molekültransfer spielt u.a. eine wichtige Rolle in der Herstellung von Vakzinen. Vakzine werden im Rahmen der aktiven Immunisierung von Menschen und Tieren zur Stimulation des Immunsystems gegen pathogene Mikroorganismen und pathogene Substanzen eingesetzt. Üblicherweise werden inaktivierte oder abgeschwächte Keime verwendet, die noch immunogen wirken. Ein geringes Gesundheitsrisiko besteht in der Aktivierung einzelner abgeschwächter Keime. Dies kann insbesondere für Patienten mit geschwächter Immunabwehr fatale Folgen haben. Wesentlich kritischer ist jedoch die Tatsache, dass für eine Vielzahl von Erkrankungen, einschließlich AIDS, derzeit keine Vakzine zur Verfügung stehen. Gentechnische Methoden sollen die Möglichkeit eröffnen, durch DNA-Transfer effiziente, hochreine synthetische Vakzine herzustellen. Der Einbau von Fremd-DNA in Pflanzen und Tieren zur Expression von Vakzinen in Lebensmitteln wird diskutiert. Prinzipiell kann eine spezielle Immunität gegen ganz bestimmte Aminosäuren-Sequenzen erzielt werden.

Der Transport von Fremd-DNA zu einer Targetzelle kann mittels spezieller Träger, z.B. kolloidaler Partikel, wie Nano- und Mikrospheren, Emulsionen und Liposomen, erfolgen. So werden derzeit virusähnliche Aggregate (VLA), in denen die zu transportierenden Moleküle von einer zweischichtigen Membran umgeben sind, erforscht.
Werden derartige kolloidale Partikel beispielsweise intravenös appliziert, so werden diese üblicherweise mit hoher Effizienz durch das retikuloendotheliale System (Kupferzellen etc.) abgefangen und können dadurch den Transfer zum eigentlichen Target nicht bewirken. Um diese unspezifische Aufnahme zu verhindern, wird die Oberfläche der Partikel z.B. durch bestimmte Beschichtungen sowie durch geeignete Partikelgröße mittels aufwendigem Partikel-Engineering verändert. Wird schließlich die Zielzelle im Zielorgan mehr oder weniger effizient und selektiv erreicht, bestehen Probleme bei der Partikelaufnahme und die Gefahr einer Vernichtung durch lysosomale Enzyme und Nukleasen.
Eine effiziente Fremd-DNA-Akkumulation im Zytoplasma oder direkt im Zellkern einer spezifischen Zielzelle ist daher wünschenswert.
Der gezielte Transfer von Molekülen, bevorzugt von DNA in vitale Zellen, erfolgt bislang
(i) mit mechanischen Verfahren, wie der Mikroinjektion und dem Beschuss mittels "Partikel-Gun",
(ii) mit Hilfe von biologischen (Viren, Bakterien etc.) und synthetisierten Träger-Molekülen oder
(iii) durch Permeabilisierung der Membran mittels elektrischer Felder (Elektroporation) oder chemischen Agenzien (z.B. mittels Toxin Streptolysin-O).
Probleme bestehen bei allen drei Methoden hinsichtlich der Effizienz des Molekültransfers und der hohen Wahrscheinlichkeit einer unbeabsichtigten letalen Wirkung, wie es der nachfolgend aufgeführte Stand der Technik belegt.

Es ist bekannt (z.B. D.J. Stephens, R. Pepperkok: The many ways to cross the plasma membrane, Proc. Nat. Acad. Sci. USA, 89 (2001) 4295-4298; D. Luo, W.M. Saltzman: Synthetic DNA delivery systems, Nature Biotechnol. 18 (2000) 33-37; L. Bildirici, P. Smith, C. Tzavelas, E. Horefti, D. Rickwood: Transfection of cells by immunoporation, Nature 405 (2000) 298) einen Transfer von Molekülen, bevorzugt von DNA in vitale Zellen, mit Hilfe von synthetisierten Träger-Molekülen und von biologischen TrägerSystemen (Viren etc.) durchzuführen.
Ebenfalls bekannt ist es, den gezielten Transfer von Molekülen durch mechanische Verfahren, wie der Mikroinjektion und der Beschuss mittels "Partikel-Gun", zu ermöglichen (z.B. M. Knoblauch et al.: A galinstan expansion femtosyringe for microinjection of eukaryotic organelles amd prokaryotes, Nature Biotechnol. 17 (1999) 906-909).
Außerdem ist der Transfer von Molekülen in vitale Zellen durch Permeabilisierung der Membran mittels elektrischer Felder (Elektroporation) oder chemischen Agenzien (z.B. Toxin Streptolysin-O) bekannt.
Bei der konventionellen gentechnologischen Vakzin-Herstellung werden typischerweise unter hohen Sicherheitsmaßnahmen Zellkulturen in Bioreaktoren mit Viren als Träger der interessierenden DNA infiziert, die anschließend inaktiviert oder abgeschwächt werden.

Der direkte Transfer einzelner Moleküle in eine speziell ausgewählte einzelne Zelle ist nur mittels mechanischer Mikroinjektion unter Verwendung dünner Glaskanülen (typischer distaler Durchmesser: 0,5 mm) durch invasive Disruption der Zellmembran möglich, verläuft jedoch ineffizient und mit hohem Schädigungspotential. Der mechanische Gentransfer mittels manueller Mikroinjektion erfordert speziell ausgebildetes Personal und stößt auf erhebliche Schwierigkeiten beim Transfer in nicht-adhärente Zellen, in Pflanzenzellen infolge der stabilen Zellwand und bei isolierten Protoplasten. Zudem bereitet die Anwesenheit des Glases im Zellinneren infolge Haftung von intrazellulären Molekülen, z.B. bestimmten Proteinen, erhebliche Probleme. Die auftretenden mechanischen Kräfte induzieren zusätzliche Destruktionseffekte.

Optische Methoden zum gezielten Molekültransfer auf der Basis fokussierter Laserstrahlung durch Mikroablation eines Membranabschnitts erfolgten bislang mittels ultravioletter Laserquellen mit Impulsbreiten im Nanosekundenbereich und hohen Energien im Mikrojoule-Bereich und Millijoule-Bereich. Laserimpulse derart langer Impulsbreite erzeugen kollaterale destruktive mechanische Effekte durch intensive photodisruptive Prozesse. Zudem ist die Anwendung von ultravioletter Strahlung auf Grund cytotoxischer und mutagener Effekte umstritten. UV-Strahlung wird auch außerhalb des Fokusbereiches von einer Vielzahl endogener Moleküle absorbiert. Die Erfolgsrate einer derartigen optischen Transfektion ist dadurch gering. Versuche zum lasergestützten Gentransfer finden seit 1984 statt (Tsukakoshi et al.: Appl. Phys. B35 (1984) 135-140). Üblicherweise werden Ultraviolett(UV)-Nanosekunden-Laser relativ hoher Impulsenergie im µl-Bereich und Einzelschussbetrieb eingesetzt. Die Transfektionsraten sind dabei sehr gering, wie in der Fachliteratur übereinstimmend von Tsukakoshi et al. (in: Appl. Phys. B35 (1984) 135-140) und Kurato et al. (in: Exp. Cell Res. 162 (1986) 372-378) über Transfektionseffizienzen von maximal 0,6 % dokumentiert ist. Dabei wurde von Tsukakoshi et al. eine Anordnung zum Transfer von Genen beschrieben, die auf einem frequenz-verdreifachten 10 Hz Nd:YAG Nanosekunden-Laser bei einer Arbeitswellenlänge von 355 nm basiert, die zudem noch mit einem He-Ne Laser als Pilotlaser ausgerüstet ist und eine Strahlablenkung durch den Einsatz von zwei Galvoscannern ermöglicht. Mittels einer sogenannten Transilluminationsvorrichtung wird unter Verwendung eines UV-Sperrfilters ein Bild der Probe auf einem TV-Monitor dargestellt und mittels Videorekorder aufgenommen. Dabei wurden zur Perforation der Zellmembran Einzelschüsse mit hohen Impulsenergien von 1 mJ eingesetzt.

Auch Stickstoff-Laser mit einer Emissionswellenlänge von 337 nm wurden bei einen Gentransfer in Pflanzenzellen eingesetzt (Weber at al.: Naturwissenschaften 75 (1988) 36). Bei UV-Laser-gestützten Gentransfers in Pflanzenembryonen wurde über eine Transormationseffizienz von 0,5 % berichtet (Greulich: Micromanipulation by light in biology and medicine, Birkhäuser Verlag, 1999).

In einer weiteren Veröffentlichung von Tao et al. (in: PNAS 84 (1987) 4180-4184) wird von UV-Belichtung mit µJ-Impulsen einer Impulsdauer von 10 ns bei einem relativ großen Bestrahlungsareal von 2,0 µm Durchmesser unter Verwendung eines inversen ZEISS-Mikroskops mit 32x Objektiv berichtet. Bei einer derartigen Vergrößerung beträgt die numerische Apertur typischerweise weniger als 0,8. Der damit verbundene relativ große Bestrahlungsspot verursachte wahrscheinlich eine erhebliche Perforation in der Membran der gleichen Größenordnung. Die Transformationseffizienz bei diesen Versuchen unter Verwendung von humanen Zellen betrug weniger als 0,3 %.

### [Hier. Einfügung S. 5a]

Insgesamt ist festzustellen, dass keine in der Praxis einsetzbaren Verfahren und Vorrichtungen bekannt sind, um Moleküle, insbesondere zur Herstellung synthetischer Vakzine, in eine speziell ausgewählte einzelne Zelle effizient und wirkungsvoll sowie ohne Schädigungen der lebenden Zelle zu transferieren.

Der Erfindung liegt die Aufgabe zugrunde, eine neue Möglichkeit zum gezielten Molekültransfer in das Innere von vitalen Zellen, insbesondere den Transfer von DNA, RNA, Peptiden, Aminosäuren und Proteinen, zu finden, die eine hoher Effizienz des Transfers erreicht und destruktive Nebeneffekte, wie z.B. eine letale Wirkung auf eine behandelte Zelle, weitgehend ausschließt.

Erfindungsgemäß wird die Aufgabe bei einem Verfahren zum Transfer von Molekülen in vitale Zellen mittels Laserstrahlung, bei dem zum Molekültransfer von DNA, RNA, Peptiden, Aminosäuren oder Proteinen gezielt eine Targetzelle durch einen gepulsten nah-infraroten (NIR-) Laserstrahl mit einer Impulsbreite im Femtosekunden-Bereich bestrahlt wird, dadurch gelöst, dass der NIR-Laserstrahl mit einem Submikrometer-Spot auf eine zelluläre Membran einer pflanzlichen, tierischen oder humanen Targetzelle fokussiert wird, wobei die Membran der Targetzelle für eine Bestrahlungsdauer von weniger als einer Sekunde durch multiple Laserimpulse mit Impulsenergien im Mikro- bis Nanojoule-Bereich bestrahlt wird, so dass die Membran lediglich transient für einen gezielten Molekültransfer von DNA, RNA, Peptiden, Aminosäuren oder Proteinen geöffnet wird.

Vorteilhaft kommen für die Laserbestrahlung multiple Laserimpulse mit einer impulsfolgefrequenz im MHz-Bereich oder höher zur Anwendung.
Vorzugsweise werden Laserimpulse mit Energien im Bereich von wenigen NanoJoule eingesetzt.
Zweckmäßig wird der Laser so eingestellt, dass im Laserspot auf der Membran der Targetzelle eine mittleren Lichtintensität im TW/cm²-Bereich erreicht wird.

Ferner sind im Stand der Technik Lösungen bekannt, die unter Verwendung von Strahlung im nahen Infrarot (NIR-Spektralbereich) mit der Methode der Fluoreszenzmikroskopie eine Bilderfassung von lebendem Zellmaterial und mit NIRlmpulslasern ein nanochirurgisches "Skalpell" zum Isolieren, Trennen und Entfernen von zellinneren (intrazellularen) Strukturen beschreiben.
So wird von TIRLAPUR et al. in The Plant Journal 20 , 3 (1999), 363-370 offenbart, dass in den Zellen einer Pflanzenwurzel (Arabidopsis) ein Farbstoff erfolgreich in eine Targetzelle eingebracht werden kann, wenn nah-infrarote Strahlung (NIR ≥ 800 nm) definierter Dauer und Leistung gezielt auf das Zentrum der Zelle fokussiert wird Dabei ist nach einer gewissen Zeit beobachtet worden, dass auch nichtbestrahlte Nachbarzellen den Farbstoff aufnehmen. Da zum Nachweis der erfolgreichen Einbringung des Farbstoffs ein Dead-Cell-Indikator (Propidiumjodid) verwendet wird, der eine Rotfärbung der Zelle bewirkt, ist jedoch mit der Indikation der Farbstoffeinbringung auch zugleich deren Zelltod besiegelt, so dass die Einbringung von Fremdstoffen in die Targetzelle nicht unter Erhaltung ihrer Vitalität erreicht wird.

Des Weiteren werden von K. KÖNIG in Histochem. Cell Biol. Bd. 114, 2 (2000) 79-92, NIR-Laser zur kontaktlosen Laserchirurgie beschrieben, wobei eine Kombination von NIR-Lasermikroskop und fokussierten, gepulst betriebenen NIR-Lasern als biomedizinisches Werkzeug für optische intrazellulare Mikromanipulation, Photochemie und Nanochirurgie, angewendet wird. Dabei wird ausgenutzt, dass ein plasmavermittelter Photoschädigungsprozess auf kleinste intrazellulare Strukturen begrenzt werden kann, um Laserschnitte mit einer minimalen Ausdehnung von 110 nm auszuführen. Zur Erhaltung der Zellvitalität werden mit dem Laserfokus ohne Einwirkung auf die Zellmembran gezielt die zu manipulierenden inneren Zellstrukturen bearbeitet, z.B. Zerlegen von Chromosomen oder Eliminieren von intrazellularen Strukturen. Gearbeitet wird mit Durchschnittsleistungen von 30-40 mW bei µs- bis ms-Einwirkungsdauer des Lasers. Das plasmavermittelte auf Photoschädigung beruhende Prinzip dieses NIR-Laserskalpells lässt eine Perforation der Zellmembran für einen Molekültransfer ohne letale Wirkungen nicht sinnvoll erscheinen.

Es weist sich als vorteilhaft für die Genauigkeit und räumlichen Begrenzung der transienten Membrandurchlässigkeit für den Molekültransfer, dass der Laserstrahl beugungsbegrenzt auf einen Submikrometer-Spot fokussiert wird.

Des Weiteren wird die obige Aufgabe zwecks einer technischen Umsetzung des Verfahrens erfindungsgemäß in einer Anordnung zum Molekültransfer in vitale Zellen mittels Laserstrahlung, bei der zum Molekültransfer vorgesehene Zellen, wie DNA, RNA, Peptiden, Aminosäuren oder Proteine, in einer extrazellulären Umgebung von Molekülen, die in das Innere einer Zelle gelangen sollen, eingelagert sind und bei der ein Impulslaser zur Erzeugung eines nah-infraroten (NIR-) Laserstrahls aus multiplen Impulsen mit Impulsdauern von weniger als 500 fs und Impulsenergien im Mikro- bis Nanojoule-Bereich vorhanden ist, der mit einer Fokussieroptik, die im Spot auf eine Lichtintensität im Bereich von Terawatt pro Quadratzentimeter einstellbar ist, mit einem Submikrometer-Spots auf die biologische Membran einer Targetzelle fokussiert ist, und Einrichtungen zur Justierung und Beobachtung der Targetzelle vorhanden sind, die einen Probentisch mit einer z-Verstelleinheit zur submikrometer-genauen Positionierung der Zellmembran gegenüber dem Laserspot sowie gegenüber dem Laserfokus enthalten, eine zusätzliche Beleuchtungsquelle, eine Abbildungsoptik, Spezialfilter und eine CCD-Kamera zum Auffinden und Justieren der Targetzelle aufweisen und zum Erfassen laserinduzierter hochlokalisierter transienter Membranveränderungen auf den vom Laser belichteten Spot auf der Zellmembran ausgerichtet sind, dadurch gelöst, dass dem Impulslaser ein schneller Shutter zur Einstellung von kurzen Bestrahlungsdauern im Bereich von Millisekunden bis zu einigen Mikrosekunden nachgeordnet ist, so dass eine begrenzte Einwirkung der multiplen Laserimpulse auf die Membran der Targetzelle lediglich eine transiente Öffnung der Membran zum Transfer von DNA, RNA, Peptiden, Aminosäuren oder Proteinen bewirkt.

Vorteilhaft weist die Beleuchtungsquelle in Kombination mit der Abbildungsoptik und CCD-Kamera zur Targetsuche, Targetpositionssteuerung und Beobachtung der transienten Membranveränderung eine Strahlung zur Durchlichtbeleuchtung, vorzugsweise weißes Licht, auf.

In einer anderen vorteilhaften Variante weist die Beleuchtungsquelle eine Strahlung für eine Fluoreszenzanregung auf, wobei damit endogene Fluorophore (Autofluoreszenz) oder vorzugsweise exogene (verabreichte) Fluorophore (z.B. fluoreszierende Membranmarker) anregbar sind.
Es erweist sich weiterhin als zweckmäßig, wenn der Laserstrahl zugleich zur zweiphotonen-angeregten Fluoreszenz eines fluoreszierenden Membranmarkers eingesetzt wird.
Vorteilhaft weist der Laserstrahl im Laserspot auf der Targetzelle eine Impulsenergie von weniger als 100 nm auf.
Für das Probenhandling hinsichtlich der Ausrichtung des Laserspots auf eine einzige Targetzelle und eine ausgewählte Membranoberfläche ist der Laserstrahl auf einen festen Punkt fokussiert, wobei die Positionierung des Laserstrahls zur Targetmembran mittels des Probentisches und die Fokussteuerung über die z-Verstelleinheit des Objektivs vorgesehen ist.

Der Shutter ist vorteilhaft zwischen einem ersten Öffnungszustand zur Bereitstellung von Nanojoule-Impulsen für die Membran-Bearbeitung und einem zweiten Öffnungszustand zur Bereitstellung von Pikojoule-Impulsen für die Beobachtung der Strahlposition umschaltbar.
Zur Detektion der Position des Laserstrahls bezüglich der Targetzelle ist die CCD-Kamera vorteilhaft mit einem Filter ausgestattet, so dass auf sie geringe von der Targetzelle oder deren Träger reflektierte oder transmittierte Anteile des Laserstrahls abgebildet werden, und weist weiterhin Rechner- und Steuerelemente zur Darstellung des Laserspotanteils simultan mit der durch die Strahlung der zusätzlichen Beleuchtungsquelle erzeugten Abbildung der Targetzelle auf.
Zweckmäßig weist die der CCD-Kamera nachgeordnete Bildverarbeitungseinheit einen Rechner mit spezieller Bildverarbeitungs-Software zur Identifizierung von Targetzellen auf.
Dabei ist der Rechner vorteilhaft zugleich mit Steuer- und Verstelleinheiten zur Ansteuerung des Probentisches verbunden, wobei die aus der Objekt-Identifizierung über Targetzellen erhaltenen Informationen zur Auswahl und Positionierung der Targetzelle sowie zur Laserfokussierung vorgesehen sind.

Die Steuer- und Verstelleinheiten sind auf Basis der vom Rechner gelieferten Objektidentifizierung zweckmäßig zum Positionieren einer ausgewählten Membran der Targetzelle im Laserfokus vorgesehen.
Weiterhin enthält die Bildverarbeitungseinheit vorteilhaft eine Bildverarbeitungssoftware, mit der der Verlauf der transienten Membranveränderungen erfassbar ist. Damit kann der erfolgreiche Molekültransfer nachgewiesen und die Suche und Bearbeitung der nächsten Targetzelle automatisiert eingeleitet werden.

Der Grundgedanke der Erfindung liegt darin, eine Membran einer vitalen Targetzelle (Prokaryot, Eukaryont) durch Laserbelichtung mit multiplen Laserimpulsen im Millijoule- oder Nanojoule-Energiebereich in einem Submikrometer-Spot über eine Bestrahlungsdauer von weniger als eine Sekunde transient für den Molekültransfer zu öffnen. Dadurch können außer der äußeren Zellmembran weiteren zellinnere Membranen, z. B. die Kernmembran, gezielt bearbeitet werden.
Es hat sich gezeigt, dass durch Anwendung einer solchen speziellen Laserbestrahlung eine laserinduzierte hochlokalisierte transiente Membranveränderung unter Aufhebung der Barrierefunktion im Bereich des Laserspots den kurzzeitigen wirkungsvollen Transfer von Molekülen in die Zelle und somit eine effiziente Transfektion ermöglichen, ohne dass dieser Molekültransfer von destruktiven Nebeneffekten begleitet wird. Die eingangs genannten störenden Nebeneffekte, wie phototoxische Effekte und geringe Transfektionseffizienz treten nicht auf.

Die Erfindung soll nachstehend anhand eines Ausführungsbeispieles näher erläutert werden. Die Zeichnungen zeigen:
- Fig. 1:: eine Prinzipansicht der erfindungsgemäßen Anordnung,
- Fig. 2:: eine Darstellung der erfolgreichen Transfektion von CHO-Zellen mit pEGFP-N1 in Form einer bildlichen Erfassung der Expression wenige Stunden nach Bestrahlung mit den intensiven Femtosekundenimpulsen mit CCD-Aufnahmen von Einphotonen- und Zweiphotonen-Fluoreszenz-Imaging sowie der NIR-Transmissionsabbildung.

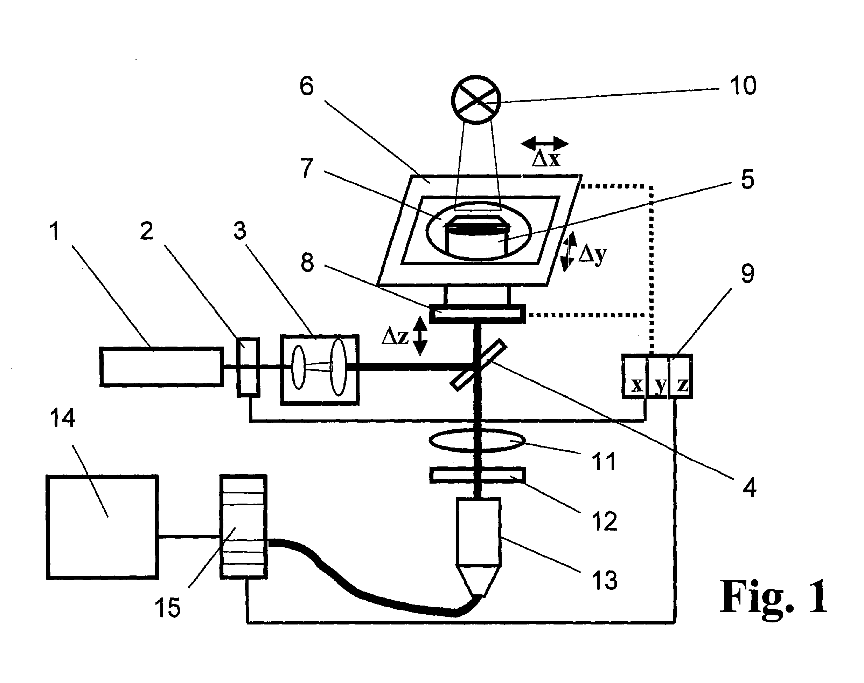

Die Erfindung wird in ihrem Verfahrensablauf - ohne Beschränkung der Allgemeinheit - unter Bezugnahme auf eine in Fig. 1 schematisch dargestellte Anordnung beschrieben.
Die Anordnung besteht in ihrem Grundaufbau aus einem Laser 1 mit vorgeordnetem Shutter 2 und Strahlaufweiter 3, einem Objektiv 5, einem motorisierten Probentisch 6 mit darauf befindlichen Targetzellen 7, der über eine z-Verstelleinheit 8 des Objektivs 5 und eine x-y-z-Verstelleinheit 9 die Targetzellen gegenüber dem vom Objektiv 5 erzeugten Laserspot beweglich lagert, einer zusätzlichen Beleuchtungsquelle 10 sowie eine CCD-Kamera 13 zur Aufnahme eines mit der Beleuchtungsquelle 10 erzeugten Abbildes der behandelten Targetzellen 7.
Als Laser 1 wird ein modensynchronisierter 80-MHz Titan-Saphir Laser in ein Laserscanning-Mikroskop eingekoppelt und mittels eines 40x-Objektivs 5 der hohen numerischen Apertur von 1.3 beugungsbegrenzt auf einen Sub-Mikrometer-Spot fokussiert. Die mittlere Leistung beträgt bei einer Impulsbreite von ca. 200 fs zunächst wenige Mikrowatt zur Beobachtung und Targetzellsuche. Mittels Scanner wurden zunächst eine Zellschicht von CHO Zellen (Chinesische Hamsterzellen), eine Zellschicht von PTK-Zellen (Rattenkänguru-Zellen) und eine Zellschicht von adulten humanen DPSC-Stammzellen aufgebracht und ein Bild anhand des transmittierten Strahls und der Detektion mittels Photomultiplier erstellt. In einzelnen Fällen kann auch das Zweiphotonen-angeregte Fluoreszenzsignal eines fluoreszierenden Membranmarkers für die Bilderstellung verwendet werden. Die Zellen befinden sich üblicherweise in einer miniaturisierten sterilen Zellkammer, die 0.5 ml Kulturmedium und 0.2 µg Plasmid DNA Vector pEGFP-N1 (4.7 kb) enthält. Dieses Plasmid bewirkt die Synthese von grün fluoreszierendem Protein.
Anschließend wird der Laserstrahl auf einen ausgewählten Submikrometer-Bereich der Membran einer einzelnen ausgewählten Zelle (Scanner im Punkt-Bestrahlungsmodus) fokussiert und die Leistung auf 50 mW bis 100 mW erhöht. Der Bereich wird mittels schnellem Strahlshutter für 16 ms bestrahlt. Nachfolgende Scans bei der reduzierten mittleren Mikrowatt-Leistung zeigten das Bestehen einer transienten Membranveränderung (Membranausstülpung) im Bereich des ausgewählten vom Laserspot getroffenen Membranbereiches der Targetzelle. Eine solche Bestrahlung bewirkt wahrscheinlich die kurzzeitige Mikroperforation der Zellmembran, durch die Plasmid in die Zelle eindringen kann.
Der Bestrahlungsmodus wurde an 200 Zellen wiederholt getestet. Typischerweise beträgt der zeitliche Aufwand für die Transfektion durch Zellauffindung, Strahlfokussierung und Bestrahlung 10 bis 15 Sekunden.

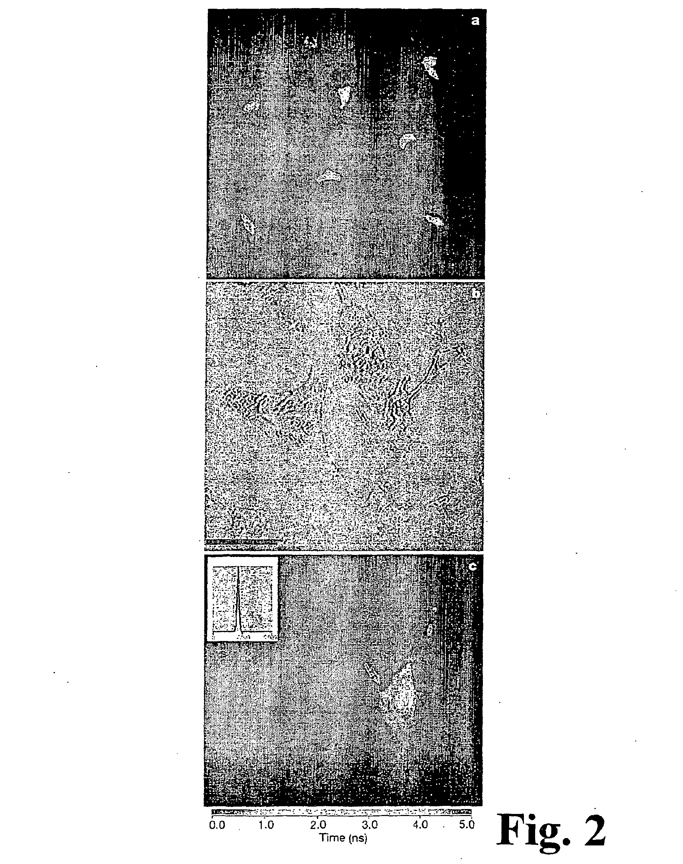

Die Integration des DNA-Plasmids und die Expression des grün fluoreszierenden Proteins wurde in situ durch stationäres und zeitaufgelöstes Zweiphotonen-Fluoreszenz-Imaging bei einer mittleren Laserleistung < 1 mW über einen Zeitraum von 72 h untersucht. Die erfolgreiche laserinduzierte Expression von EGFP wurde durch die Messung der mittleren Fluoreszenzlebensdauer von -2.4 ns bestätigt. Unabhängig vom Zelltyp konnte eine Transfektionsrate sowie eine Expressionsrate von mehr als 90 % erzielt werden, wie die Darstellungen von Fig. 2 eindrucksvoll belegen.

Die erfindungsgemäße Anordnung zum effizienten Molekültransfer in eine einzelne vitale Zelle enthält vorzugsweise einen modensynchronisierten Femtosekundenlaser hoher Folgefrequenz, der im Wellenlängenbereich zwischen 700 nm und 1200 nm (NIR) emittiert und über ein Objektiv 5 hoher numerischer Apertur (größer 0,8) einen starr fixierten und beugungsbegrenzt auf einen Submikrometer-Spot fokussierten Laserstrahl aus multiplen Nanojoule-Laserimpulsen mit einer Lichtintensität im TW/cm²-Bereich zur transienten Aufhebung der Barrierefunktion von biologischen Membranen im Bereich des Laserspots bereitstellt. Weiterhin besteht die Anordnung aus einem schnellen Shutter 2, der Bestrahlungsdauern im Mikro- und Millisekunden-Bereich unter Einsatz von Nanojoule-Impulsen sowie Pikojoule-Impulse zur Targeteinstellung realisiert, einer zusätzlichen Beleuchtungsquelle 10, Abbildungsoptik 11, Spezialfilter 12 und CCD-Kamera 13 für das Auffinden und Justieren des Targets sowie zur Erfassung laser-induzierter hochlokalisierter transienter Membran-Veränderungen, einem vorzugsweise motorisierten Probentisch 6 mit Submikrometer-Genauigkeit, einer z-Verstelleinheit 8, einer Bildverarbeitungseinheit 15 mit Objekt-Identifizierung und Steuerungsmodulen zum automatisierten Transfer von DNA, RNA und Proteinen in vitale Einzelzellen.

Vorzugsweise kommt ein Femtosekunden-Laserscanning-Mikroskop zum Einsatz, wie es für Anwendungen im Bereich der Zellbiologie bekannt ist (siehe z.B. Denk et al. Science 248 (1990) 73). Im Gegensatz zu einer üblicherweise über einen (kostenintensiven) Galvoscanner erfolgenden Strahlablenkung (mit speziellen Scanoptiken, Strahlführungen und Ansteuerungen), wird zum Abzurastern der Proben gemäß der Erfindung der Strahl des Femtosekundenlasers mittels Strahlaufweiter 3, Umlenkeinheit 4 und Objektiv 5 auf einen fixen Punkt lokalisiert. Das Scan- und Fokussierregime wird allein über den motorisierten Probentisch 6 und die Verstelleinheiten 8 und 9 realisiert.

In einer bevorzugten Ausführung von Fig. 1 wird als Laser 1 für den effizienten Molekültransfer ein gepulster Festkörperlaser hoher Strahlgüte (TEM₀₀-Mode) mit einer Emissionswellenlänge von 800 nm, hoher Folgefrequenz (mit typischen Werten um 80 MHz), einer Impulsdauer von weniger als 300 fs und einer Impulsenergie von wenigen Nanojoule (<100 nJ) eingesetzt. Der Laserstrahl kann mit einem schnellen Shutter 2 mit minimalen Schaltzeiten im Mikrosekundenbereich nahezu verlustfrei für die Bearbeitung der Targetzelle 7 freigegeben oder für die Detektion der Laserstrahlposition in der Targetzelle 7 zwischen 95% und 99% geblockt werden, bevor er auf den Aufweiter 3 trifft. Über den nachfolgenden Umlenkspiegel 4 (mit einem NIR-Reflexionsvermögen zwischen 90 % und 99 %) wird der Strahl dann auf die Fokussieroptik 5 mit einer numerischen Apertur größer 0,8, typischerweise 1,2, gelenkt und dadurch auf einen beugungsbegrenzten Submikrometer-Spot innerhalb der auf dem Probentisch 6 befindlichen Probe 7 fokussiert.
Die Probe, z.B. eine vitale Zelle, - als Zielobjekt vereinfacht: Targetzelle 7 - befindet sich zweckmäßigerweise in einer Miniatur-Zellkammer mit mindestens einem ca. 170 µm dicken Glasfenster. Die Targetzelle 7 ist typischerweise von einem Medium umgeben, welches auch zu transferierende Moleküle, z.B. bestimmte DNA-Plasmide, enthält. Der Laserstrahl wird durch das Glasfenster auf einen Membranabschnitt der Targetzelle 7 fokussiert. Dieser Membranabschnitt kann die Zellmembran, eine Zellwand, die Membran einer Organelle oder die Kernmembran darstellen.
Die Fokusebene kann mittels einer piezogetriebenen z-Verstelleinheit 8 in der Tiefe (z-Richtung) mit einer Genauigkeit von weniger als 100 nm verändert werden. Alle drei Richtungen x, y, z am Positioniertisch 6 sind mit der zugehörigen Verstelleinheit 9 mit Submikrometergenauigkeit (z.B. mit integriertem Joystick) verstellbar.
Die Beleuchtungsquelle 10 geringer Lichtintensität gewährleistet eine Abbildung der Targetzelle 7 auch während der Laserbestrahlung durch die Kombination mit der Abbildungsoptik 11, Kurzpass-Filter 12, der eine starke Abschwächung der Laserstrahlung bewirkt, und CCD-Kamera 13. Die tatsächliche Position des Laserspots wird neben der Visualisierung von transmittiertem Licht der Beleuchtungsquelle 10 derart aufgenommen, dass der an der Targetzelle 7 und/oder deren Glasträger reflektierte Laserstrahl mit hoher räumlicher Präzision als heller, nicht überstrahlter Laserspot zusammen mit dem durch die Beleuchtungsquelle 10 erzeugten Bild der Targetzelle 7 im zentralen Teil des Monitors 14 dargestellt werden kann. Ein Rechner, vorzugsweise ein PC, als Bildverarbeitungseinheit 15 mit Steuer- und Regeleinheit und einem Bildanalyse-Programm zur Objekt-Identifizierung (Pattern Recognition System) ermöglicht die Erkennung der Target-Membran sowie die automatisierte Verschiebung des Probentisches 6 und der z-Verstelleinheit 8 derart, dass ein Teil der Targetmembran und der Laserfokus übereinstimmen. Bei voll oder teilweise automatischem Betrieb wird unmittelbar nach Targeteinstellung der Shutter 2 angesteuert und für Bestrahlungszeiten von weniger als einer Sekunde eine Vielzahl von Nanojoule-Laserimpulsen auf eine Membran der Targetzelle 7 appliziert. Simultan und kurz nach Bestrahlung kann bei erfolgreicher Laserbestrahlung eine transiente Veränderung der Membran in Form einer Ausstülpung auf dem Monitor 14 beobachtet werden. Üblicherweise halten diese Membranveränderungen einige Sekunden bis einige Minuten an. Unmittelbar nach Bestrahlung ist die Membran für Moleküle durchlässig und ermöglicht den Transfer von DNA, RNA und Proteinen besonders effektiv. Wird die transiente Veränderung durch das Bildananlyse-Programm erfasst, wird ein neues Target, üblicherweise eine neue Targetzelle 7, durch Betätigung der Verstelleinheit 9 mittels Steuermodul automatisch eingestellt und der beschriebene Vorgang wiederholt. Wird keine transiente Veränderung der Membran im Bereich des Bestrahlungsfeldes erkannt, erfolgt die Einstellung auf eine benachbarte Membranregion der gleichen Zelle und eine erneute Laserbestrahlung. Die Bestrahlungsquelle 10 arbeitet im einfachsten Fall mit weißem Licht. Anstelle von Weißlicht kann sie aber auch eine Fluoreszenzanregungsstrahlung emittieren, welche die Detektion von fluoreszierenden Membranmarkern mittels CCD-Kamera 13 ermöglicht. Dabei kann der Laser 1 selbst als Fluoreszenzanregungsquelle durch Zweiphotonen-Effekte genutzt werden. Zudem kann die beschriebene Anordnung auch in ein Mikroskop integriert werden.
Als Targetmembran können nicht nur Zellmembran oder Zellwand sondern auch intrazelluläre Membranen, wie Kernmembran und Mitochondrien-Membran, verstanden werden.

Fig. 2 dokumentiert die erfolgreiche Transfektion von CHO-Zellen mit pEGFP-N1 und die bildliche Erfassung der Expression wenige Stunden nach Bestrahlung mit den intensiven Femtosekundenpulsen durch NIR-Transmissionsaufnahme, Zweiphotonen-Fluoreszenz-Imaging und CCD-Darstellung. Dabei zeigen die Teilabbildungen (Maßstab: Bar = 25 µm):
a: ein reales EGFP-Fluoreszenzbild mittels CCD-Kamera nach Einphotonen-Blauanregung. Deutlich erkennbar sind eine Vielzahl von fluoreszierenden Zellen nach erfolgreicher Laser-Transfektion.
b: ein NIR-Transmissionsbild mit Pfeil, der die einzelnen laserbearbeiteten Zellen anzeigt und
c: ein Zweiphotonen-Fluoreszenzlebensdauer-Bild, in dem die einzelne fluoreszierende Zelle nach Laserbearbeitung deutlich erkennbar ist. Umgebende Zellen, die nicht mit den intensiven Laserimpulsen bestrahlt wurden, fluoreszieren nicht signifikant. Die kalkulierte Fluoreszenzlebensdauer von 2.4 ns entspricht dem zu erwartenden Wert für das grün fluoreszierende Protein (GFP).

### Bezugszeichenliste

- 1: Laser
- 2: Shutter
- 3: Aufweiter
- 4: Umlenkspiegel
- 5: Objektiv
- 6: Probentisch
- 7: Targetzelle (Probe)
- 8: z-Verstelleinheit
- 9: Verstelleinheit
- 10: (zusätzliche) Beleuchtungsquelle
- 11: Abbildungsoptik
- 12: Kurzpass-Filter
- 13: CCD-Kamera
- 14: Monitor
- 15: Bildverarbeitungseinheit

## Patentansprüche

1. Verfahren zum in vitro transfer von DNA, RNA, Peptiden, Aminosäuren oder Proteinen in vitale Zellen mittels Laserstrahlung, bei dem zum Molekültransfer von DNA, RNA, Peptiden, Aminosäuren oder Proteinen gezielt eine Targetzelle durch einen gepulsten nah-infraroten (NIR-) Laserstrahl mit einer Impulsbreite im Femtosekunden-Bereich bestrahlt wird, **dadurch gekennzeichnet, dass**
- der NIR-Laserstrahl mit einem Submikrometer-Spot auf eine zelluläre Membran einer pflanzlichen, tierischen oder humanen Targetzelle fokussiert wird, wobei
- die Membran der Targetzelle für eine Bestrahlungsdauer von weniger als einer Sekunde durch multiple Laserimpulse mit Impulsenergien im Mikro- bis Nanojoule-Bereich bestrahlt wird, so dass die Membran lediglich transient für einen gezielten Molekültransfer von DNA, RNA, Peptiden, Aminosäuren oder Proteinen geöffnet wird.

2. Verfahren gemäß Anspruch 1, **dadurch gekennzeichnet, dass**
multiple Laserimpulse mit einer Impulsfolgefrequenz im MHz-Bereich oder höher zur Anwendung kommen

3. Verfahren gemäß Anspruch 1, **dadurch gekennzeichnet, dass**
multiple Laserimpulse mit Energien im Bereich von wenigen Nanojoule eingesetzt werden.

4. Verfahren gemäß Anspruch 1, **dadurch gekennzeichnet, dass**
multiple Laserimpulse mit einer mittleren Lichtintensität im TW/cm²-Bereich verwendet werden.

5. Verfahren gemäß Anspruch 1, **dadurch gekennzeichnet, dass**
der Laserstrahl beugungsbegrenzt auf den Submikrometer-Spot fokussiert wird.

6. Anordnung zum Molekültransfer von DNA, RNA, Peptiden, Aminosäuren oder Proteinen in vitale Zellen mittels. Laserstrahlung, bei der zum Molekültransfer vorgesehene Zellen, in einer extrazellulären Umgebung von Molekülen, die in das Innere einer Zelle gelangen sollen, eingelagert sind und bei der
- ein Impulslaser zur Erzeugung eines nah-infraroten (NIR-) Laserstrahls aus multiplen Impulsen mit lmpulsdauern von weniger als 500 fs und Impulsenergien im Mikro- bis Nanojoule-Bereich vorhanden ist, der mit einer Fokussieroptik, die im Spot auf eine Lichtintensität im Bereich von Terawatt pro Quadratzentimeter einstellbar ist, mit einem Submikrometer-Spots auf die biologische Membran einer Targetzelle fokussiert ist, und
- Einrichtungen zur Justierung und Beobachtung der Targetzelle vorhanden sind, die einen Probentisch mit einer z-Verstelleinheit zur submikrometer-genauen Positionierung der Zellmembran gegenüber dem Laserspot sowie gegenüber dem Laserfokus enthalten, eine zusätzliche Beleuchtungsquelle, eine Abbildungsoptik, Spezialfilter und eine CCD-Kamera zum Auffinden und Justieren der Targetzelle aufweisen und zum Erfassen laserinduzierter hochlokalisierter transienter Membranveränderungen auf den vom Impulslaser belichteten Spot auf der Membran der Targetzelle ausgerichtet sind,
**dadurch gekennzeichnet, dass**
- dem Impulslaser ein schneller Shutter zur Einstellung von kurzen Bestrahlungsdauern im Bereich von Millisekunden bis zu einigen Mikrosekunden nachgeordnet ist, so dass eine begrenzte Einwirkung der multiplen Laserimpulse auf die Membran der Targetzelle lediglich eine transiente Öffnung der Membran zum Transfer von DNA, RNA, Peptiden, Aminosäuren oder Proteinen bewirkt.

7. Anordnung nach Anspruch 6, **dadurch gekennzeichnet, dass**
die zusätzliche Beleuchtungsquelle in Kombination mit der Abbildungsoptik und CCD-Kamera zur Targetsuche, Targetpositionssteuerung und Beobachtung der transienten Membranveränderung eine Strahlung zur Durchlichtbeleuchtung, vorzugsweise weißes Licht, aufweist.

8. Anordnung nach Anspruch 6, **dadurch gekennzeichnet, dass**
die Beleuchtungsquelle eine Strahlung für eine Fluoreszenzanregung aufweist, wobei damit endogene Fluorophore (Autofluoreszenz) oder vorzugsweise exogene (verabreichte) Fluorophore (z.B. fluoreszierende Membranmarker) anregbar sind.

9. Anordnung nach Anspruch 6, **dadurch gekennzeichnet, dass**
der Laserstrahl zugleich zur zweiphotonen-angeregten Fluoreszenz von exogenen Fluorophoren, vorzugsweise fluoreszierenden Membranmarkern, eingesetzt ist.

10. Anordnung nach Anspruch 6, **dadurch gekennzeichnet, dass**
der Laserstrahl auf einen festen Punkt fokussiert ist, wobei die Positionierung des Spots zur Targetmembran mittels des Probentisches und die Fokussteuerung über die z-Verstelleinheit des Probentisches vorgesehen ist.

11. Anordnung nach Anspruch 6, **dadurch gekennzeichnet, dass**
der Laserstrahl im Laserspot auf der Targetzelle eine Impulsenergie von weniger als 100 nJ aufweist.

12. Anordnung nach Anspruch 11, **dadurch gekennzeichnet, dass**
der Shutter zwischen einem ersten Öffnungszustand zur Bereitstellung von Nanojoule-Impulsen für die Membran-Bearbeitung und einem zweiten Öffnungszustand zur Bereitstellung von Pikojoule-Impulsen für die Beobachtung der Strahlposition umschaltbar ist.

13. Anordnung nach Anspruch 6, **dadurch gekennzeichnet, dass**
die CCD-Kamera mit einem Filter versehen ist, so dass auf sie geringe von der Targetzelle oder von einem Träger der Targetzelle reflektierte oder transmittierte Anteile des Laserstrahls abgebildet werden, und eine Bildverarbeitungseinheit zur Darstellung des Laserspots simultan mit der durch die Strahlung der zusätzlichen Beleuchtungsquelle erzeugten Abbildung der Targetzelle aufweist.

14. Anordnung nach Anspruch 6, **dadurch gekennzeichnet, dass**
die der CCD-Kamera nachgeordnete Bildverarbeitungseinheit einen Rechner mit spezieller Bildverarbeitungs-Software zur Identifizierung von Targetzellen aufweist.

15. Anordnung nach Anspruch 14, **dadurch gekennzeichnet, dass**
der Rechner mit Steuer- und Verstelleinheiten zur Ansteuerung des Probentisches verbunden ist, wobei die Informationen der Bildverarbeitung und Objekt-Identifizierung zur Auswahl und Positionierung der Targetzelle sowie zur Laserfokussierung vorgesehen sind.

16. Anordnung nach Anspruch 15, **dadurch gekennzeichnet, dass**
die Steuer- und Verstelleinheiten auf Basis der vom Rechner gelieferten Objekt-Identifizierung zum Positionieren einer ausgewählten Membran der Targetzelle im Laserfokus vorgesehen sind.

17. Anordnung nach Anspruch 14, **dadurch gekennzeichnet, dass**
die Bildverarbeitungseinheit eine Bildverarbeitungssoftware enthält, mit der der Verlauf der transienten Membranveränderungen erfassbar ist.

## Claims

1. A method for *in vitro* transfer of DNA, RNA, peptides, amino acids, or proteins into vital cells by means of laser irradiation, in which, for the molecular transfer of DNA, RNA, peptides, amino acids, or proteins, a target cell is irradiated in a targeted manner by a pulsed near-infrared (NIR) laser beam with a pulse width in the femtosecond range,
**characterised in that**
- the NIR laser beam with a submicron spot is focused on a cellular membrane of a plant, animal, or human target cell, whereby
- the membrane of the target cell is irradiated for an irradiation duration of less than a second by multiple laser pulses with pulse energies in the microjoule to nanojoule range, so that the membrane is opened only transiently for a targeted molecular transfer of DNA, RNA, peptides, amino acids, or proteins.

2. A method according to claim 1, **characterised in that**
multiple laser pulses with an pulse frequency in the MHz range or higher are used.

3. A method according to claim 1, **characterised in that**
laser pulses with energies in the range of a few nanojoules are used.

4. A method according to claim 1, **characterised in that**
multiple laser pulses with a mean light intensity in the TW/cm² range are used.

5. A method according to claim 1, **characterised in that**
the laser beam is focused on the submicron spot in a diffraction-limited manner.

6. An arrangement for the molecular transfer of DNA, RNA, peptides, amino acids, or proteins into vital cells by means of laser irradiation, with which cells intended for the molecular transfer are stored in an extra-cellular environment of molecules that are to reach the interior of a cell, and in which
- a pulse laser is present for the generation of a near-infrared (NIR) laser beam of multiple pulses with a pulse width of less than 500 fs and pulse energies in the microjoule to nanojoule range, which is focused with a submicron spot onto a biological membrane of target cell with a focusing optic that can be adjusted in a spot to a light intensity in a range of terawatts per square centimeter, and
- devices are present for adjusting and monitoring the target cells that include a specimen table with a z-adjustment unit for submicron accurate positioning of the cell membrane with respect to the laser spot and the laser focus, that have an additional illumination source, imaging optics, a special filter, and a CCD camera for locating and adjusting the target cells, and which are aligned with spot on the membrane of the target cell illuminated by the pulse laser to capture laser-induced, highly-localised, transient membrane changes,
**characterised in that**
- a fast shutter is arranged subsequent to the pulse laser for adjustment of short irradiation periods in the range from a milliseconds to a few microseconds, so that the limited effect of the multiple laser pulses on the membrane of the target cells causes only a transient opening of the membrane for the transfer of DNA, RNA, peptides, amino acids, or proteins.

7. An arrangement according to claim 6, **characterised in that**
the additional illumination source, in combination with the focusing optics and the CCD camera for the target search, target-position control, and monitoring of the transient membrane changes has a beam for transillumination, preferably, a white light.

8. An arrangement according to claim 6, **characterised in that**
the illumination source has a beam for fluorescence excitation, where, with this, endogenic fluorophores (auto-fluorescence) or, preferably, exogenic (administered) fluorophores (eg fluorescing membrane markers) can be excited.

9. An arrangement according to claim 6, **characterised in that**
the laser beam is used at the same time for two-photon excitation of exogenic fluorophores, preferably fluorescing membrane markers.

10. An arrangement according to claim 6, **characterised in that**
the laser beam is focused on a fixed point, whereby the positioning of the spot to the target membrane is provided by means of the specimen table and the focus control, using the z-adjustment unit of the specimen table.

11. An arrangement according to claim 6, **characterised in that**
the laser beam in the laser spot on the target cell has an pulse energy of less than 100 nJ.

12. An arrangement according to claim 11, **characterised in that**
the shutter can be switched between a first opening state for supplying the nanojoule pulses for the membrane processing and a second opening state for supplying the picojoule pulses for monitoring the beam position.

13. An arrangement according to claim 6, **characterised in that**
the CCD camera is supplied with a filter, so that small portions of the laser beam, reflected or transmitted from the target cell or from a carrier of the target cell, are imaged, and having an image-processing unit for simultaneous representation of the laser spot with the image of the target cell created by the irradiation of the addition illumination source.

14. An arrangement according to claim 6, **characterised in that**
the image processing unit arranged downstream from the CCD camera has a computer with special image-processing software for identification of the target cells.

15. An arrangement according to claim 14, **characterised in that**
the computer is connected with control and adjustment units for controlling the specimen table, whereby the information from the image processing and from the object identification of target cells is provided for selecting and positioning the target cells and for focusing the laser.

16. An arrangement according to claim 15, **characterised in that**
control and adjustment units are provided for positioning a selected membrane of the target cell in the laser focus on the basis of the object identification delivered from the computer.

17. An arrangement according to claim 14, **characterised in that**
the image-processing unit contains image-processing software with which the course of the transient membrane changes can be captured.

## Revendications

1. Procédé pour le transfert *in vitro* d'ADN, d'ARN, de peptides, d'acides aminés ou de protéines, dans des cellules vivantes à l'aide d'un rayonnement laser dans lequel pour le transfert de molécules d'ADN, d'ARN, de peptides, d'acides aminés ou de protéines, de manière ciblée, une cellule cible est irradiée par un rayon laser du proche infrarouge (NIR) pulsé avec une durée d'impulsion de l'ordre des femtosecondes, **caractérisé en ce que**
- le rayonnement laser NIR est focalisé à l'aide d'un spot sub-micrométrique sur une membrane cellulaire d'une cellule cible végétale, animale ou humaine, où
- la membrane de la cellule cible est irradiée pendant une période de rayonnement de moins d'une seconde par plusieurs impulsions laser avec des énergies d'impulsion de l'ordre des micro- à nanojoules, de sorte que la membrane est ouverte de manière simplement transitoire pour un transfert moléculaire ciblé d'ADN, d'ARN, de peptides, d'acides aminés ou de protéines.

2. Procédé selon la revendication 1, **caractérisé en ce que** les impulsions laser multiples sont utilisées avec une fréquence des impulsions située dans la gamme des MHz ou plus.

3. Procédé selon la revendication 1, **caractérisé en ce que** les impulsions laser multiples sont mises en oeuvre avec des énergies situées dans la gamme de quelques nanojoules.

4. Procédé selon la revendication 1, **caractérisé en ce que** les impulsions laser multiples sont utilisées avec une intensité lumineuse moyenne dans la gamme des TW/cm².

5. Procédé selon la revendication 1, **caractérisé en ce que** le rayon laser est focalisé avec diffraction limitée, en le spot sub-micrométrique.

6. Dispositif pour le transfert de molécules d'ADN, d'ARN, de peptides, d'acides aminés ou de protéines, dans des cellules vivantes à l'aide d'un rayonnement laser, dans lequel les cellules prévues pour le transfert moléculaire sont disposées dans un environnement extracellulaire des molécules, qui doivent parvenir à l'intérieur d'une cellule et dans lequel
- un laser à impulsion pour la production d'un rayonnement laser du proche infrarouge (NIR) par plusieurs impulsions avec des durées d'impulsion de moins de 500 fs et des énergies d'impulsion de l'ordre des micro- et des nanojoules, est présent, lequel est focalisé avec une optique de focalisation, qui peut être ajusté au niveau du spot, à une intensité lumineuse dans la gamme des térawatts par centimètre carré, avec un sport sub-micrométrique sur la membrane biologique d'une cellule cible, et
- des dispositifs pour ajuster et observer la cellule cible, sont présents, qui contiennent un plateau d'échantillon, avec une unité ajustable en Z pour le positionnement à précision sub-micrométrique de la membrane cellulaire par rapport au spot laser ainsi que par rapport au foyer laser, une source supplémentaire d'illumination, une optique de reproduction, un filtre spécial et une caméra CCD pour trouver et ajuster la cellule cible et pour saisir les modifications transitoires, très localisées, induites par le laser, de la membrane sur le spot illuminé de l'impulsion laser sur la membrane de la cellule cible, **caractérisé en ce que**
- le laser à impulsion est muni d'un shutter rapide pour l'ajustement de courtes durées de rayonnement dans la gamme des millisecondes à quelques microsecondes, de sorte qu'une action limitée des impulsions laser multiples sur la membrane de la cellule cible effectue simplement une ouverture transitoire de la membrane pour le transfert d'ADN, d'ARN, de peptides, d'acides aminés ou de protéines.

7. Dispositif selon la revendication 6, **caractérisé en ce que** la source d'illumination supplémentaire présente, en combinaison avec l'optique de représentation et la caméra CCD, un rayonnement pour l'illumination par transmission, de préférence de lumière blanche, pour la recherche de la cible, le réglage du positionnement de la cible et l'observation de la modification transitoire de la membrane.

8. Dispositif selon la revendication 6, **caractérisé en ce que** la source d'illumination présente un rayonnement pour une excitation par fluorescence, où les fluorophores endogènes (autofluorescence) ou de préférence, les fluorophores exogènes (administrés) (par exemple marqueur membranaire fluorescent) sont excitables.

9. Dispositif selon la revendication 6, **caractérisé en ce que** le rayon laser est mis en oeuvre en même temps, pour la fluorescence excitée par deux photons de fluorophores exogènes, de préférence des marqueurs membranes fluorescents.

10. Dispositif selon la revendication 6, **caractérisé en ce que** le rayon laser est focalisé sur un point fixe, où le positionnement du spot sur la membrane cible est prévu à l'aide du plateau d'échantillon et de la commande de focalisation par l'unité ajustable en Z du plateau d'échantillon.

11. Dispositif selon la revendication 6, **caractérisé en ce que** le rayon laser présente dans le spot laser sur la cellule cible, une énergie d'impulsion de moins de 100 nJ.

12. Dispositif selon la revendication 11, **caractérisé en ce que** le shutter peut être commuté entre un premier état d'ouverture pour la mise en place des impulsions nanojoules pour le traitement de la membrane et un deuxième état d'ouverture pour la mise en place des impulsions picojoules pour l'observation de la position du rayonnement.

13. Dispositif selon la revendication 6, **caractérisé en ce que** la caméra CCD est équipée d'un filtre, de sorte que les faibles quantités du rayon laser, réfléchies ou transmises par la cellule cible ou le support de la cellule cible, sont formées sur celui-ci et elle présente une unité de traitement d'image pour la représentation du spot laser simultanément à la représentation produite par le rayonnement de la source d'illumination supplémentaire de la cellule cible.

14. Dispositif selon la revendication 6, **caractérisé en ce que** l'unité de traitement d'image agencée après la caméra CCD présente un ordinateur avec logiciel spécial de traitement d'image pour l'identification de la cellule cible.

15. Dispositif selon la revendication 14, **caractérisé en ce que** l'ordinateur est relié à des unités de commande et d'ajustement pour le réglage du plateau d'échantillon, où les informations du traitement d'image et d'identification de l'objet sont destinées au choix et au positionnement de la cellule cible ainsi que pour la focalisation du laser.

16. Dispositif selon la revendication 15, **caractérisé en ce que** les unités de commande et d'ajustement sont destinées au positionnement d'une membrane choisie de la cellule cible dans le foyer du laser, sur base de l'identification d'objet offerte par l'ordinateur.

17. Dispositif selon la revendication 14, **caractérisé en ce que** l'unité de traitement d'image contient un logiciel de traitement d'image, avec lequel on peut saisir le développement des modifications transitoires de la membrane.
